(19) Europäisches Patentamt / European Patent Office / Office européen des brevets

(11) **EP 2 016 891 B1**

(12) **EUROPEAN PATENT SPECIFICATION**

(45) Date of publication and mention of the grant of the patent:
**19.10.2016 Bulletin 2016/42**

(51) Int Cl.:
*A61B 5/00* *(2006.01)*      *G01N 21/47* *(2006.01)*
*G01N 21/17* *(2006.01)*      *G06F 17/14* *(2006.01)*

(21) Application number: **08010634.7**

(22) Date of filing: **11.06.2008**

(54) **Medical apparatus for obtaining information indicative of internal state of an object based on physical interaction between ultrasound wave and light**

Medizinische Vorrichtung zum Erhalt von Informationen, die den internen Zustand eines Objekts basierend auf der physischen Interaktion einer Ultraschallwelle und Licht indizieren

Appareil médical pour obtenir des informations indiquant l'état interne d'un objet basé sur une interaction physique entre des ondes à ultrasons et la lumière

(84) Designated Contracting States:
**DE FR GB**

(30) Priority: **11.06.2007 JP 2007154562**

(43) Date of publication of application:
**21.01.2009 Bulletin 2009/04**

(73) Proprietor: **Olympus Corporation**
**Tokyo 192-8507 (JP)**

(72) Inventor: **Igarashi, Makoto**
**Tokyo 151-0072 (JP)**

(74) Representative: **von Hellfeld, Axel**
**Wuesthoff & Wuesthoff**
**Patentanwälte PartG mbB**
**Schweigerstrasse 2**
**81541 München (DE)**

(56) References cited:
**EP-A- 1 810 610      WO-A-98/50781**
**WO-A-02/059580      WO-A-2006/090298**
**JP-A- 2005 224 399      US-B1- 6 738 653**

**Description**

Background of the Invention

(The Field of the Invention)

The present invention relates to a medical apparatus and method for observing the internal state of an object to be examined, and in particular to, a medical apparatus that has the capability of optically acquiring and observing information indicative of the internal state of an object to be examined by using a physical interaction between ultrasound wave and light.

(Related Art)

In recent medical imaging, it is significant to obtain high-precision tomographic images of an object and lower invasiveness in acquiring those images. From this point of view, optical tomographic imaging has attracted attention. As this optical tomographic imaging, there have been known optical CT (computed tomography), optical coherence tomography (OCT), and photoacoustic tomography.

Of these techniques, the optical CT utilizes near-infrared light of a wavelength ranging from 700 nm to 1200 nm, which is comparatively weakly influenced by scattering in a living body. Therefore, the optical CT enables to obtain tomograms of deep parts in a living body, such as up to several centimeters under the mucous membrane.

The OCT, which utilizes low-coherence interference, enables to obtain tomographic images of a living body up to a depth of about 2 mm with high resolution (several $\mu$m to several tens of $\mu$m) in short time. The OCT is a technology that has already been put into practice in diagnosing retinopathy in an ophthalmic field, and has attracted very keen interest in the medical world.

Although the optical CT can provide information on a deep part of a living body, its spatial resolution is as low as several millimeters. In contrast, it is difficult for the OCT to enable observation at a depth of about 2 mm or more under the mucous membrane and to provide good quality images of tumor tissue, such as cancer. This is because the optical coherence is greatly disturbed by the influence of strong absorption or multiple scattering in the deep part of a living body and tumor tissue.

Considering this situation, other techniques for obtaining internal information of an object has been provided. One of such techniques is shown by Japanese Patent Laid-open Publication No. 2000-88743 (patent reference (1)) and reference "L. V. Wang and G. Ku, "Frequency-swept ultrasound-modulated optical tomography of scattering media", Optics Letters, Vol.23, Issue 12, pp. 975-977(1998)" (non-patent reference (1)). These techniques shows that ultrasound wave and light are radiated into a target portion of an object in order to detect which amount the light is modulated by the ultrasound in the target portion.

Another technique is provided by Japanese Patent Laid-open Publication No. 10-179584 (parent reference (2)). In this case, a pulsed ultrasound wave and electromagnetic radiation are radiated into a target portion of a living body in order to detect electromagnetic radiation scattering backward in the target portion.

In the living body, it is known that light scattering characteristics are altered, in particular, by the morphological changes of a biological tissue, such as the alteration in the concentration of nuclear chromatin accompanying the canceration of a tumor and the alteration in the spatial distribution of nuclei. Thus, if information Indicative of changes in the light scattering characteristics in a target portion of a living body is obtained, it is possible to detect structural changes of a tissue such a cancer tissue. It is useful to adopt, as such information, changes in the real part of the complex refractive index in the target portion.

However, the techniques provided by the patent reference (1) and the non-patent reference (1) are directed to only obtaining Information showing changes in the optical absorption characteristics of a target portion. Hence it is difficult to detect the structural and morphological changes of, for example, a cancer tissue.

In addition, the technique provided by the patent reference (2) is dedicated to detecting the backward-scattered electromagnetic radiation. From this technique, it is still unknown how to detect changes in the light scattering characteristics in the living body.

WO 2006/090298 A1 discloses an apparatus for imaging an object of interest comprising an ultrasound device adapted to emit ultrasonic waves of multiple frequencies onto the object of interest. An electromagnetic radiation detector is adapted to detect electromagnetic fluorescence radiation emitted by a fluorescence donor and acceptor provided to an object of interest, wherein the donor and acceptor allow energy to be transferred from a donor to an acceptor to generate fluorescence light, in order to monitor donor and acceptor pairs within a given distance of each other.

WO 98/50781 A1, disclosing the preamble of claim 1 and claim 14, teaches a system that detects laser light that is transmitted through a turbid medium. Light diffused by the sample and the turbid medium is modulated by an ultrasonic frequency sweep signal having a frequency that varies linearly with time. The light intensity at different frequencies is detected by a photomultiplier tube (PMT) and mapped to a location in the zone of interest to obtain a one-dimensional image of the turbid medium along the ultrasonic axis.

WO 02/059580 A1 is a method of imaging an absorption and/or scattering image of a region of interest inside a scattering medium using a mathematical model representative of a relation between the distribution of the intensity and phase of electromagnetic radiation components scattered from a medium and an attenuation factor dependent on scattering and absorption coefficients of the medium. In this method, an ultrasound wave and laser light are directed into a region of interest, and a detector detects the signal resulting from the interaction between the two waves, in which the frequency/phase of the light is shifted by the frequency/phase of the ultrasound. The configuration maps the location of the interaction of the light waves and the ultrasound waves along the Z-axis by the phase of the ultrasound pulses of a given frequency. The presence of an absorbing agent in the scattering medium is determined from the change in the intensity distribution of the frequency/phase shifted light signals. The scattering component of the light is determined by analyzing the phase change of the signal.

US 6,738,653 B1 discloses an apparatus for determining oxygenation levels of hemoglobin in a probed region of the body by irradiating a region with diffuse light, the frequency of which is shifted with ultrasound waves of a given frequency focused at the same region, and determining the light absorption at the focal region of the ultrasound. The ratio of the ultrasound modulated light signal with the intensity of the non-modulated light signal yields the absorption characteristics. Further, the scattering and absorption coefficients are determined as factors of the measured intensity of the modulated light.

JP 2005/224399 A discloses that a light signal is passed through an ultrasonic transducer and then made incident on a measurement sample, and that the amplitude of the signal is measured over time.

Summary of the Invention

The present invention has been made in light of the problems as described above, and an object of the present invention is to provide a medical apparatus and a method of detecting information indicative of an internal state of an object to be examined that are able to capture changes in the light scattering characteristics which are caused due to structural and morphological changes of a tissue in deeper target portions of an object and to easily detect information indicating such changes. This problem is solved by a medical apparatus according to claim 1 and a method of detecting information indicative of an internal state of an object to be examined according to claim 15.

In order to achieve the above object, a medical apparatus and method according to the invention of claim 1 or claim 14 is disclosed.

For example, the ultrasound wave gradually changes its frequency over time. In particular, it is preferred that the frequency of the ultrasound wave gradually increases over time. The ultrasound wave may be continuous or pulsed.

Thus, the medical apparatus according to the present invention is able to easily capture changes in the light scattering characteristics, that is, structural and morphological changes of the tissue, in a deeper target portion inside an object.

Brief Description of the drawings

In the accompanying drawings:

Fig. 1 is a block diagram exemplifying an outline of a biological observation apparatus according to a first embodiment of the present invention;
Fig. 2A exemplifies the waveform of ultrasound wave to be radiated from an ultrasound transducer;
Fig. 2B details a start part of the ultrasound wave shown in Fig. 2A;
Fig. 2C details an end part of the ultrasound wave shown in Fig. 2A;
Fig. 2D exemplifies another waveform of ultrasound wave to be radiated from the ultrasound transducer;
Fig. 3 exemplifies calculated results obtained by a signal processor;
Fig. 4 is a flowchart showing part of the processes executed by a personal computer in the first embodiment;
Fig. 5 is an illustration pictorially explaining a two-dimensional scan in the first embodiment;
Fig. 6 is a block diagram showing a modification (a first modification) of the biological observation apparatus according to the first embodiment;
Fig. 7 exemplifies calculated results obtained in the first modification;
Fig. 8 is a block diagram showing another modification (a second modification) of the biological observation apparatus according to the first embodiment;
Fig. 9 shows a detailed configuration of an optical coupler adopted by the second modification;
Fig. 10 exemplifies the configuration of an end part of an fiber adopted by the second modification;
Fig. 11 is a block diagram exemplifying an outline of a biological observation apparatus according to a second embodiment of the present invention;

Fig. 12 is a block diagram showing a modification (a third modification) of the biological observation apparatus according to the second embodiment; and

Fig. 13 is a block diagram showing another modification (a fourth modification) of the biological observation apparatus according to the second embodiment.

Detailed Description of the Preferred Embodiments

Hereinafter, various embodiments of the present invention will now be described in connection with the accompanying drawings.

(First embodiment)

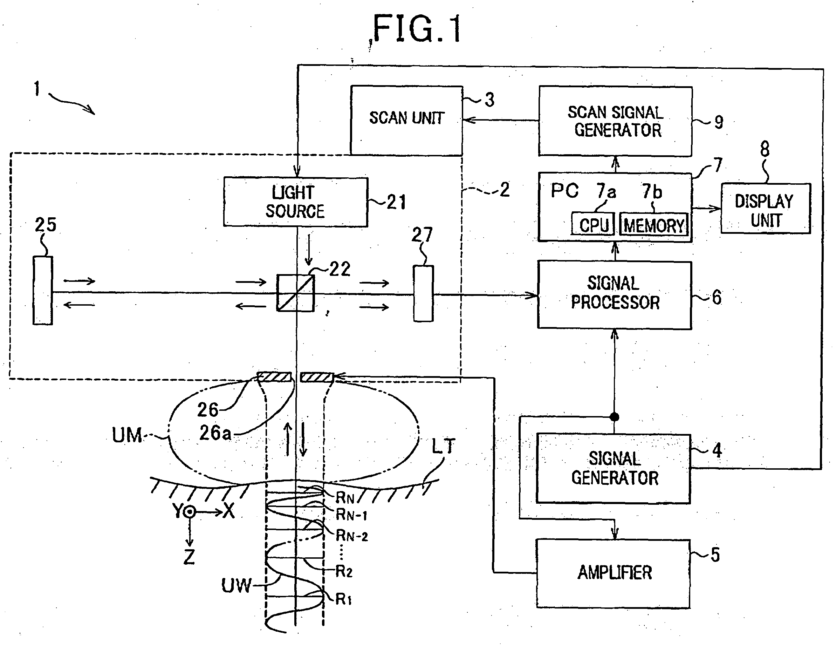

Referring to Figs. 1 - 5, a medical apparatus according to a first embodiment of the present invention will now be described. In the present embodiment, the medical apparatus is reduced into practice as a biological observation apparatus which carries out ultrasound modulated optical tomography (UMOT).

Fig. 1 outlines a biological observation apparatus 1. This biological observation apparatus 1 comprises, as shown in Fig. 1, a radiation/reception unit 2 which radiates ultrasound wave and light toward a living tissue (body tissue) LT serving as an object to be examined and receives light produced by the reflection and scattering of the radiated light from the living tissue LT (hereinafter, the reflected and scattered light is referred to as "object light"). Moreover, the biological observation apparatus 1 comprises a scan unit 3, a signal generator 4, an amplifier 5, a signal processor 6, a personal computer (hereinafter, called PC) 7, a display unit 8, and a scan signal generator 9.

The scan unit 3 is configured to, in response to a scan signal issued from the scan signal generator 9, positionally change the radiation/reception unit 2 (that is, the scan position) and, during the positional scan changes, radiate ultrasound wave and light.

The signal generator 4 produces a drive signal to make the radiation/reception unit 2 output the ultrasound wave consisting of continuous ultrasound wave whose frequencies successively change, and outputs the produced drive signal to the amplifier 5 and the signal processor 6. In addition, at a predetermined timing immediately after the radiation of the ultrasound wave from the radiation/reception unit 2, the signal generator 4 outputs a timing signal to a light source 21 provided in the unit 2.

The amplifier 5 includes a power amplifier. This amplifier 5 amplifies the power of a drive signal outputted from the signal generator 4, and provides the amplified drive signal to the radiation/reception unit 2.

The radiation/reception unit 2 is provided with, in addition to the foregoing light source 21, a half mirror 22, a reference mirror 25, an ultrasound transducer 26 with an opening 26a formed at its center, and a light detector 27.

The light source 21 has a laser, which emits light which can enter into a living tissue LT, and condenser lens, though not shown. At the predetermined timing decided by the output of the timing signal from the signal generator 4, the light source 21 radiates pulsed light, which is emitted from the laser light source, toward the half mirror 22. Incidentally, the light source which emits light which enters into the living tissue LT is not always limited to the foregoing laser light source. By way of example, the light source may be a xenon lamp, a halogen lamp, an LED (Light-Emitting Diode), or a SLD (Super Luminescent Diode). In the present embodiment, the light emitted from the light source 21 may be continuous light, not limited to the pulsed light.

Of the light which has traveled from the light source 21, the half mirror 22 reflects part of that light so that the reflected light is radiated toward the mirror 25 and allows the remaining part of that light to be transmitted toward the ultrasound transducer 26. The light which has traveled from the half mirror 22 to the reference mirror 25 is reflected by the reference mirror 25, and then made to enter the half mirror 22 as reference light. The light which has traveled from the half mirror 22 to the ultrasound transducer 26 passes through the opening 26a, before being radiated toward the living tissue LT.

In the present embodiment, a space between (the ultrasound transducer 26 of) the radiation/reception unit 2 and the living tissue LT, is filled with an ultrasound transmissive medium UM, which is for example water.

Furthermore, the ultrasound transducer 26 functions as an ultrasound wave generator. This ultrasound transducer 26 is driven in response to the drive signal from the signal generator 4 and radiates an ultrasound wave toward the living tissue LT along the axis of light passing the opening 26a. The ultrasound wave has a frequency of several MHz to several dozen MHz. This ultrasound wave travels through the inside of the living tissue LT as a cyclic compressional wave of which frequency gradually changes over time. In the present embodiment, the ultrasound wave is radiated toward the living tissue LT without being converged by a convergence lens such as an acoustic lens. But such a convergence means may be used to converge the ultrasound wave so that the converged ultrasound wave is radiated to the living tissue LT.

Thus the light that has been radiated by the radiation/reception unit 2 and passed the half mirror 22 is reflected and scattered at density maximized (i.e., higher density) portions inside the living tissue LT. The ultrasound wave locally maximizes tissue densities within the living tissue LT. That is, the density maximized local positions are produced. The reflected (and scattered) light enters, as object light, the half mirror after passing the opening 26a.

Incidentally, the radiation/reception unit 2 according to the present embodiment may be provided with a light modulator and an oscillator to drive the light modulator, where both members are arranged between the half mirror 22 and the reference mirror 25 and between the half mirror 22 and the ultrasound transducer 26, respectively. The light modulator modulates the light using a frequency greatly lower than the frequency of the light. Hence, this allows the radiation/reception unit 2 to improve the S/N of video signals outputted to the display unit 8, in cooperation with the foregoing structure of the unit 2.

The half mirror 22 allows the reference light coming from the reference mirror 25 to interfere with the object light coming from the ultrasound transducer 26, so that interference light, which is caused by interference between the two fluxes of light, is radiated toward the light detector 27.

The light detector 27 applies heterodyne detection to the interference light coming from the half mirror 22, and converts the detected interference light into an interference signal, which is an electric signal, to output the interference signal to the signal processor 6.

The signal processor 6 is provided with, for example, a spectrum analyzer or a digital oscilloscope, which functions as a light spectrum acquiring member. This signal processor 6 inputs the interference signal outputted from the radiation/reception unit 2 and the drive signal outputted from the signal generator 4 in order to acquire a frequency distribution of the interference light and a frequency distribution of the ultrasound wave outputted from the ultrasound transducer 26. Further, using a Doppler shift amount (corresponding to a frequency modulated amount) according to the spectral distribution of the interference light and the frequency components of the ultrasound wave, the signal processor 6 calculates scattering components of the object light and/or absorption components of the object light which correspond to intensities of the scattering components. The signal processor 6 outputs pieces of information showing the calculated scattering components and/or the absorption components to the PC 7.

The PC 7 comprises a CPU (central processing unit) 7a which performs various types of calculation and processing and a memory 7b which stores various data and others. In the memory 7b, programs necessary for the various types of calculation and processing carried out by the CPU 7a are previously stored as source codes. When being activated, the CPU 7a reads out the programs from the memory 7b and performs in sequence the programs, step by step. When the CPU 7a executes the programs, the PC 7 realizes desired calculation functions. Practically, the PC 7 allows the CPU 7a to perform calculation based on the information showing the scattering components and/or the absorption components outputted from the signal processor 6. Hence image data are produced. In addition, the PC 7 relates the produced image data to scan positional information which shows positions residing within a range where the scan can be performed by the scan unit 3, and the image data arid the scan positional information, which are related to each other, are stored in the memory 7b. Moreover, the PC 7 determines whether or not the current scan position that gives the image data is an end position of the scan rage controlled by the scan unit 3. When it is determined that the current scan position is not the end, the scan signal generator 9 is commanded to change the scan position and radiate the ultrasound wave and light. The PC 7 also determines whether or not the image data for one frame have been stored in the memory 7b. When the determination for this storage is affirmative, the image data is converted to image signals to be outputted to the display unit 8.

In the present embodiment, the processes carried out by the CPU 7a of the PC 7 is not always limited to the output of the image signals to the display unit 8, in which the image signals depend on only the scattering components of the object light. Alternatively, such processes may be the output of image signals depending on the absorption components of the object light to the display unit 8. Still alternatively, image signals of the scattering components and absorption components of the object light may be outputted from the PC 7 to the display unit 8 in parallel with each other or in an integrated manner.

The scan signal generator 9, which is under the control of the PC 7, changes the scan position, with which the scan signals to radiate the ultrasound wave and light is provided to the scan unit 3.

The operations of the biological observation apparatus 1 according to the present embodiment will now be described.

First of all, an operator powers up each part of the biological observation apparatus 1, and positions the ultrasound transducer 26 of the radiation/reception unit 2 such that the ultrasound wave and light are radiated in the Z-axis direction shown in Fig. 1 (i.e., the depth direction of the living tissue LT). Concurrently, a space between the ultrasound transducer 26 and the living tissue LT is filled with the ultrasound transmissive medium UM.

The operator then turns on switches, which are mounted in a not-shown operation device, to issue a command for radiating the ultrasound wave from the ultrasound transducer 26 to the living tissue LT.

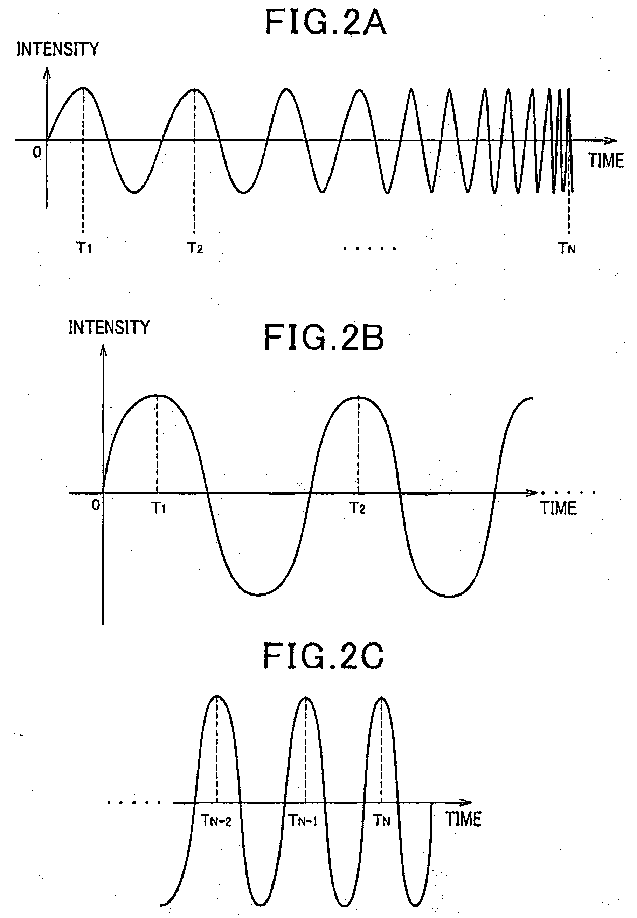

In response to the command, the signal generator 4 generates a drive signal to the ultrasound transducer 26 by way of the amplifier 5, where the drive signal is for radiating, toward the living tissue TL, predetermined ultrasound waves having a waveform shown in Fig. 2A, for example. Fig. 2B details a beginning part of the waveform shown in Fig.

2A, while Fig. 2C details an end part of the waveform shown in Fig. 2A.

Practically, as shown in Figs. 2A to 2C, the ultrasound wave has frequencies which becomes higher as time elapses (that is, each cyclic time becomes shorter) and has intensities which are maximized N times at the same one scan position at different timings. In Fig. 2A, such maximized timings are shown as times $T_1$, $T_2$, ..., $T_N$. Thus the ultrasound wave is a continuous wave formed by continuously repeating, N cycles, at each scan position, each of waveforms of frequencies $f_{us1}$, $f_{us2}$, ..., $f_{usN}$ ($f_{us1} < f_{us2} <$, ..., $< f_{usN}$). The frequencies $f_{us1}$, $f_{us2}$, ..., $f_{usN}$ are decided primarily depending on the characteristics of the ultrasound transducer 26.

Accordingly, the ultrasound wave UW radiated from the ultrasound transducer 26 (refer to Fig. 1) travels inside the living tissue LT in its depth direction as a longitudinal compressional wave from its lower-frequency wave parts (i.e., longer wavelengths) in sequence. Hence, the frequencies of the ultrasound wave UW entering the living tissue become higher over time (i.e., the wavelengths become shorter). In the living tissue LT, the ultrasound wave UW applies pressure to the internal tissue depending on its degrees of compression decided by its intensity (i.e., amplitude), which will give local compression to the tissue so that the tissue density is locally changed. This results in that, as shown in Fig. 1, the densities of the living tissue LT are locally maximized (i.e., made higher) at each of Z-axis (depth directional) directional positions (portions) in the living tissue LT, where such positions spatially correspond to the maximum intensities of the ultrasound wave UW which is transmitted inside the living tissue LT.

Those locally compressed tissue portions is greater in density from the other portions, so that such locally density-maximized tissue portions are able to reflect and scatter light. In Fig. 1, these local density-maximized tissue portions are shown as wave fronts $R_1$, $R_2$, ..., $R_N$ of the ultrasound wave UW (hereinafter referred to as ultrasound wave fronts). At a time instant when the radiation of one ultrasound wave UW has just been completed, the ultrasound wave fronts $R_1$, $R_2$, ..., $R_N$ are spatially located along the ultrasound transmission direction (the Z-axis direction) in sequence. The light is much faster in transmission speed than the ultrasound wave. Thus, from a viewpoint of the light, it is sensed such that the ultrasound wave fronts $R_1$, $R_2$, ..., $R_N$ are nearly stopped from traveling.

Meanwhile the signal generator 4 performs predetermined processes, where a timing when the ultrasound wave fronts $R_N$ are produced by the ultrasound wave UW radiated into the living tissue LT (that is, a timing immediately after completing the output of the ultrasound wave UW at each scan position) is detected and a timing signal showing the timing is produced. This timing signal is outputted to the light source 21 of the radiation/reception unit 2.

In response to the timing signal, a pulsed light is radiated from the light source 21 toward the half mirror 22. This radiated light has a frequency $f_L$ and is radiated through the opening 26a in the Z-axis direction (the depth direction in the living tissue LT) via the components including the half mirror 22 and the reference mirror 25. In the living tissue LT, the light encounters each of the plurality of ultrasound wave fronts $R_1$, $R_2$, ..., $R_N$ produced inside the living tissue LT, resulting in that the light is partly reflected (including scattering) by each wave front while the remaining is partly transmitted therethrough in the depth direction. In other words, during the travel of the light, the reflection and transmission are carried out at each of the plurality of ultrasound wave fronts $R_1$, $R_2$, ..., $R_N$. The reflected pulsed light, which is from each of the ultrasound wave fronts $R_1$, $R_2$, ..., $R_N$, is returned to the half mirror 22 via the opening 26a of the ultrasound transducer 26 as an object light (reflected light) that has been subjected to Doppler shift (i.e., frequency modulation) of a frequency $f_{d1}$ (, $f_{d2}$, ..., $f_{dN}$).

In the half mirror 22, the returned object light is made to interfere with the reference light coming form the reference mirror 25. This makes it possible to send, to the light detector, an interference light from which the component of the frequency $f_L$ is cancelled out, which frequency $f_L$ is originated from the light source 21.

In the light detector 27, the interference light is heterodyne-detected, and the detected interference light is converted to an electric interference signal to be supplied to the signal processor 6.

In the signal processor 6, the interference signal is subjected to detection of a spectral distribution. The spectral distribution of the interference light presents values at frequencies which are nearly the same as the foregoing frequencies $f_{d1}$, $f_{d2}$, ..., $f_{dN}$ showing the Doppler shift amounts (i.e., frequency modulated amounts). In addition, in the signal processor 6, using the drive signal outputted from the signal generator 4, the spectral distribution (of which frequencies are $f_{us1}$, $f_{us2}$, ..., $f_{usN}$) of the ultrasound wave emitted from the ultrasound transducer 26.

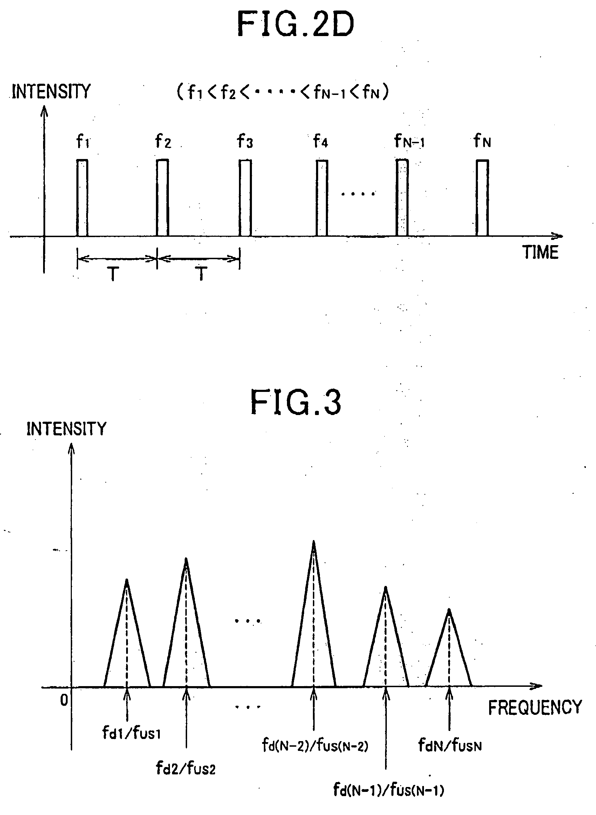

Further, the signal processor 6 also functions as a calculator, where the frequencies $f_{d1}$, $f_{d2}$, ..., $f_{dN}$ showing the Doppler shift amounts and the ultrasound frequencies $f_{us1}$, $f_{us2}$, ..., $f_{usN}$ are subjected to calculation for various physical quantities. These quantities include frequency ratios "$f_{d1}/f_{us1}$, $f_{d2}/f_{us2}$, ..., $f_{dN}/f_{usN}$" serving as scattering components of the object light P and absorption components which correspond to intensities of the scattering components of the object light at each frequency ratio. Therefore, at an operator's desired observation region, it is possible for the signal processor 6 to acquire, at the same time, living-body information from N-piece local portions in the Z-axis direction (i.e., the depth direction). Fig. 3 exemplifies a result calculated by the signal processor 6.

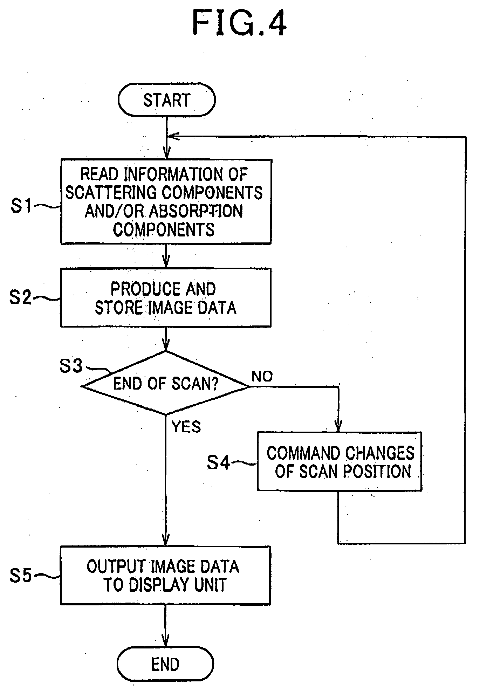

Meanwhile, the PC 7 performs a predetermined process on the execution of the CPU 7a, where information of the scattering components and absorption components given from the signal processor 6 is read in (step S1 in Fig. 4). Then, the PC 7 uses the read-in information to produce image data, and stores, into the memory 7b, the produced Image data related to scan positional information showing positions within a range to be scanned by the scan unit 3 (step S2).

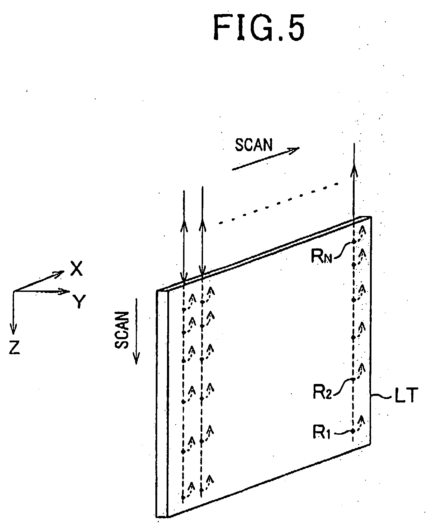

The PC 7 makes reference to information indicating a predetermined desired two- or three-dimensional scan range in order to determine if or not the current position is the end position (end part) of the scan range (step S3). The information of the scan rage is pre-given by the operator and, for example, previously stored in the memory 7b. When it is determined that the current scan position is not the end position of the scan range (NO at step S3), the PC 7 controls the scan signal generator 9 so as to radiate the ultrasound wave and the light, while the scan position is changed, radiation by radiation, in the X-axis and/or Y-axis directions (step S4). After this, the processing in the PC 7 is returned to step S1, the foregoing process is repeated. Fig. 5 pictorially shows how to perform a two-dimensional scan along for example the XZ section through repetition of the foregoing process.

On the other hand, when the PC 7 determines the current scan position is the end position of the two- or three-dimensional scan range (YES at step S3), that is, when it is determined that all image data for one frame for display has been stored in the memory 7b, the image data are converted to video signals to be outputted to the display unit 8 (step S5). Thus the display unit 8 is able to display a tomographic image of the living tissue LT residing in the operator's desired observation lesion.

As stated, according to the biological observation apparatus 1 according to the present embodiment, it is possible to easily acquire, by the one-time scan, scattering and/or observed components from the N-piece portions in the depth direction of the living tissue LT. Based on the acquired information, the two- and three-dimensional images showing the internal state of a desired portion of the object can be provided at faster speeds. In this way, the biological observation apparatus 1 according to the present embodiment is able to easily, in a shorter period of time, determine changes in the optical scattering characteristics and optical absorption characteristics in a target portion located deeply (i.e., deeper than that in the OCT diagnosis) in the object.

In addition, as shown in Fig. 2A, the present biological observation apparatus 1 radiates the ultrasound wave of which frequencies gradually increase over time. That is, the frequencies of a wave part traveling at first into the body are lower (the wavelengths are longer), and then its frequencies become higher. The lower the frequencies of the ultrasound wave, the longer its travel distance in the depth direction inside the living body. Thus, at a time instant immediately after the radiation of the ultrasound wave have been completed, there is provided an instantaneous situation where, as illustrated in Fig. 1, the ultrasound wave fronts $R_1$, $R_2$, ..., $R_N$ with larger optical refraction indices are arranged in the depth direction. In other words, there are produced the local regions where changes in the optical refractive index are enhanced by the ultrasound wave in the living body. Since the changes in the refractive index are different between medically normal parts and lesions, the changes in the refractive index can be detected, by way of example, as differences in the Doppler frequency.

In this way, by radiating the light one time when the wave fronts are arranged in the depth direction, it is possible to obtain the reflected light from each of the wave fronts due to the fact that the light travels through each wave front with transmission and reflection and is greatly faster than the ultrasound wave. By making the frequency of the ultrasound wave increase gradually over time, the radiation timing of the light can be set easily. This makes it possible to acquire the object light from each ultrasound wave front by radiating the light only one time at each scan position, making it easier to acquire the data about structural and morphological changes of the tissue in a deeper target portion of the object.

By the way, the ultrasound wave emitted from the ultrasound transducer 26 is not always limited to that shown in Fig. 2A, where the frequency increases little by little over time. Alternatively, the frequency may be decreased gradually over time.

In addition, the ultrasound wave emitted from the ultrasound transducer 26 may be a pulsed ultrasound wave as shown in Fig. 2D, not necessarily confined to the continuous ultrasound wave shown in Fig. 2A. To be specific, as shown in Fig. 2D, the pulse repetition time T is constant, but the frequencies $f_1$, $f_2$, ..., $f_{N-1}$, $f_N$ of the respective pulses of a pulse train are changed as being $f_1 < f_2 <, ..., < f_{N-1}, < f_N$. Incidentally, the pulse train shown in Fig. 2D may be such that its frequencies decrease gradually over time.

Furthermore, in the foregoing embodiment, the ultrasound wave and the light both are in parallel to the Z-axis direction, but this is not always a definitive structure. For example, any one of the two radiations may be oblique to the Z-axis direction.

<First modification>

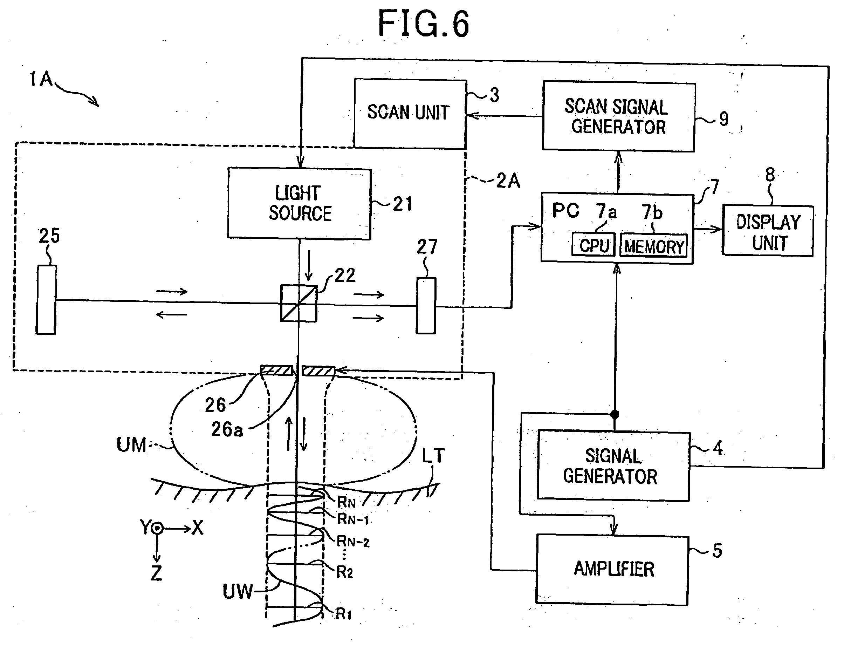

Another modification will now be explained. To obtain advantages similar to the above, the biological observation apparatus 1 shown in Fig. 1 may be modified into a biological observation apparatus 1A shown in Fig. 6. In this modification, the processes carried out by the signal processor 6 in the foregoing embodiment are carried out alternatively by the CPU 7a of the PC 7 in the similar way as the foregoing.

In the descriptions on the present modification and the following embodiment and modifications, the components which are identical or similar to those in the first embodiment will be given the same reference numerals for sake of simplified or omitted explanation.

The biological observation apparatus 1A shown in Fig. 6 comprises a radiation/reception unit 2A as well as the scan unit 3, signal generator 4, amplifier 5, PC 7, display unit 8, and scan signal generator 9, which serves as essential components.

In the present configuration of this biological observation apparatus 1A, the foregoing interference signal is directly provided from the light detector 27 to the PC 7.

Assume that DC (direct current) components of the interference signal are denoted as $I_{dc,1}$, $I_{dc,2}$, ..., $I_{dc,N}$ and AC (alternating current) component amplitudes are denoted as $I_{ac,1}$, $I_{ac,2}$, ..., $I_{ac,N}$. In this case, a light intensity $I(t)$ based on the interference signal can be shown by the following formula (1):

$$I(t) = I_{dc,1} + I_{ac,1} \cos\left[2\pi f_{d1} t + \phi_1\right]$$

$$+ I_{dc,2} + I_{ac,2} \cos\left[2\pi f_{d2} t + \phi_2\right]$$

$$+ \dots$$

$$+ I_{dc,N} + I_{ac,N} \cos\left[2\pi f_{dN} t + \phi_N\right] \quad \cdot \cdot \cdot \quad (1),$$

wherein $\phi$ depicts a phase difference.

The CPU 7a of the PC 7 applies Fourier transformation to the light intensity $I(t)$ shown by the formula (1), so that the following formula (2) can be obtained.

$$F(\omega) = \int_{-T}^{+T} \left\{ \sum_{i=1}^{N} I_{dc,i} + I_{ac,i} \cos(\omega_i t + \phi_i) \right\} e^{-j\omega t} dt$$

$$= \sum_{i=1}^{N} \frac{2 I_{dc,i} \sin \omega T}{\omega} + \sum_{i=1}^{N} I_{ac,i} \cos\phi \left\{ \frac{\sin(\omega_i - \omega)T}{(\omega_i - \omega)} + \frac{\sin(\omega_i + \omega)T}{(\omega_i + \omega)} \right\}$$

$$+ j \sum_{i=1}^{N} I_{ac,i} \sin\phi \left\{ \frac{\sin(\omega_i - \omega)T}{(\omega_i - \omega)} + \frac{\sin(\omega_i + \omega)T}{(\omega_i + \omega)} \right\} \quad \cdot \cdot \cdot \quad (2),$$

wherein $\omega_i = 2\pi f_{di}$.

The CPU 7a, which also functions as a light spectrum acquiring member, extracts real-number components (i.e., intensity) in the formula (2) as values showing the spectral distribution of the interference light.

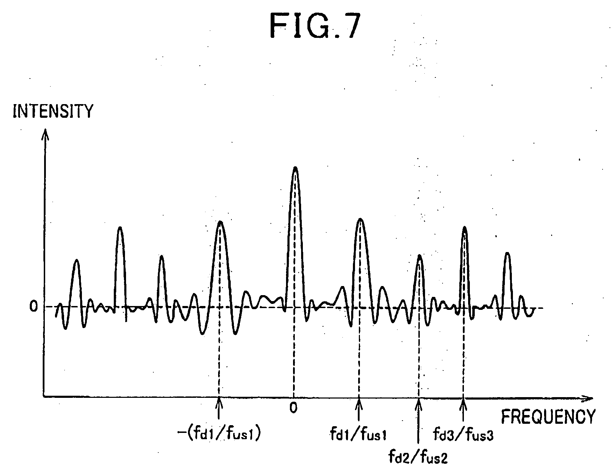

Then the CPU 7a also functions as a calculator, where frequencies $f_{d1}$, $f_{d2}$, ..., $f_{dN}$ indicating Doppler shift amounts depending on the spectral distribution of the interference light and frequencies of the ultrasound wave from the ultrasound transducer 26, $f_{us1}$, $f_{us2}$, ..., $f_{usN}$ are treated to calculate the ratios $f_{d1}/f_{us1}$, $f_{d2}/f_{us2}$, ..., $f_{dn}/f_{usN}$, serving as the scattering components of the object light. In addition, the CPU 7a calculates the intensities at the respective ratios as the absorption components of the object light. A result calculated by the CPU 7a is exemplified in Fig. 7.

In this way, at an operator's desired portion to be observed, it is possible to acquire, at a time, pieces of body information at N-piece parts in the Z-axis direction within the living body LT. That is, the biological observation apparatus 1A shown in Fig. 6 also obtains the similar advantages to those gained by the foregoing apparatus 1. In addition, this apparatus 1A has no signal processor, thereby simplifying the apparatus compared to the foregoing apparatus 1.

<Second modification>

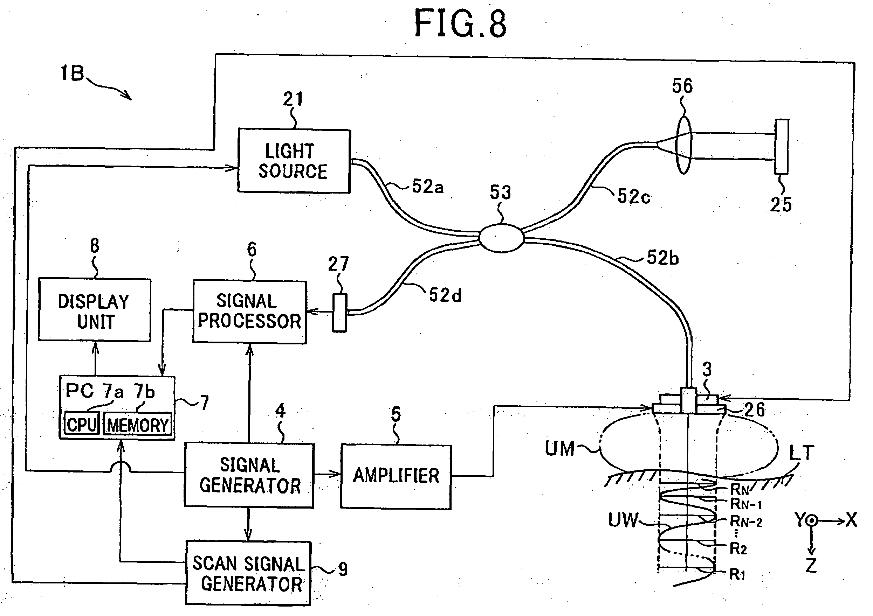

The biological observation apparatus 1 shown in Fig. 1 can be modified into a biological observation apparatus 1B shown in Fig. 8, where the apparatus 1B, which functions as an object information analyzing apparatus, comprises optical fibers and an optical coupler.

Practically, a biological observation apparatus 1B shown in Fig. 8 comprises, as essential components, optical fibers 52a to 52d, an optical coupler 53, and a collimating lens 56, in addition to the scan unit 3, signal generator 4, amplifier 5, signal processor 6, PC7, display unit 8, scan signal generator 9, light source 21, reference mirror 25, ultrasound transducer 26, and light detector 27.

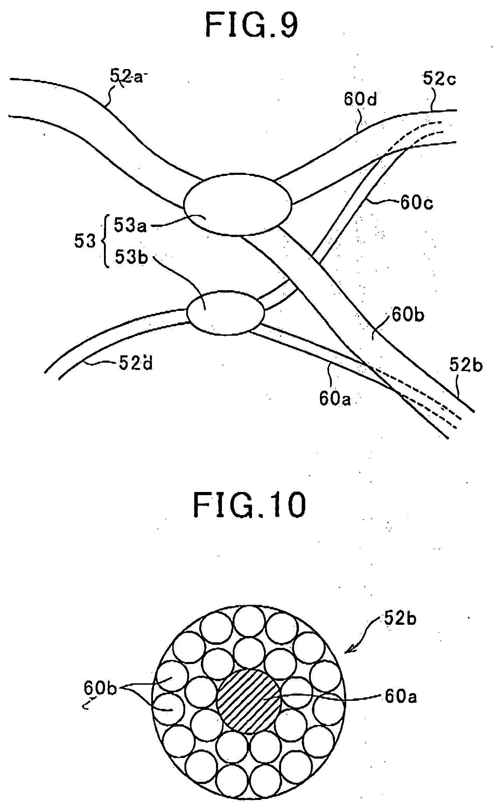

The optical coupler 53 comprises, as shown in Fig. 9, a first coupler 53a and a second coupler 53b. The first coupler 52a has one end connected to the light source 21 and the other end connected to the first coupler 53a, as shown in Figs. 8 and 9.

The optical fiber 52b comprises, as shown in Fig. 9, a light-receiving fiber bundle 60a and a light-sending fiber bundle 60b. The fiber bundle 60a has one end connected to the second coupler 53b and the other end connected to an opening (for example, though not shown in Fig. 8, the opening 26a) formed at the center of the ultrasound transducer 26 in such a manner that the end is inserted therethrough. Meanwhile the fiber bundle 60b has one end connected to the first coupler 53a and the other end connected to an opening (for example, though not shown in Fig. 8, the opening 26a) formed at the center of the ultrasound transducer 26 in such a manner that the end is inserted therethrough. Both other ends of the respective fiber bundle 60a and 60b are arranged at the opening of the ultrasound transducer 26 as illustrated in Fig. 10.

The optical fiber 52c also comprise, as shown in Fig. 9, a light-receiving fiber bundle 60c and a light-sending fiber bundle 60d. The fiber bundle 60c has one end connected to the second coupler 53b and the other end arranged at a predetermined position where the light comes in from the collimating lens 56. Moreover the fiber bundle 60d has one end connected to the first coupler 53a and the other end arranged at a predetermined position where the light can be radiated to the collimating lens 56.

The optical fiber 52d has, as shown in Figs. 8 and 9, one end connected to the second coupler 53b and the other end connected to the light detector 27.

As described, in this biological observation apparatus 1B, the light generated by the light source 21 is radiated to both the living body LT via the optical fiber 52a, the first coupler 53a, and the fiber bundle 60b and the collimating lens 56 via the optical fiber 52a, the first coupler 53a, and the fiber bundle 60d.

The light which enters the collimating lens 56 is converted to parallel-flux light and radiated to the reference mirror 25. This light is reflected by the reference mirror 25, the reflected light is made to pass through the collimating lens 56 again, and is radiated to the fiber bundle 60c as reference light. This reference light, incident on the fiber bundle 60c, is then radiated to the second coupler 53b.

The light radiated into the living tissue LT is partly reflected in sequence at the respective ultrasound wave fronts $R_1$, $R_2$, ..., $R_N$ produced therein, where the object light (reflected light) subjected to Doppler shift (frequency modulation) of frequencies $f_{d1}$, $f_{d2}$, ..., $f_{dN}$ is generated as described. This object light is made to enter the fiber bundle 60a and then radiated to the second coupler 53b serving as a light reception section.

In the second coupler 53b, the object light interferes with the reference light coming from the fiber bundle 60c, with the result that interference light with frequency component $f_L$ cancelled out is produced and radiated to the light detector 27. The frequency component $f_L$ is originated from the light from the light source 21.

The processes applied to the interference light which has come to the light source 21 are the same or similar as or to those in the first embodiment. Hence, the biological observation apparatus 1B shown in Fig. 8 is able to have the advantages similar to the foregoing.

(Second embodiment)

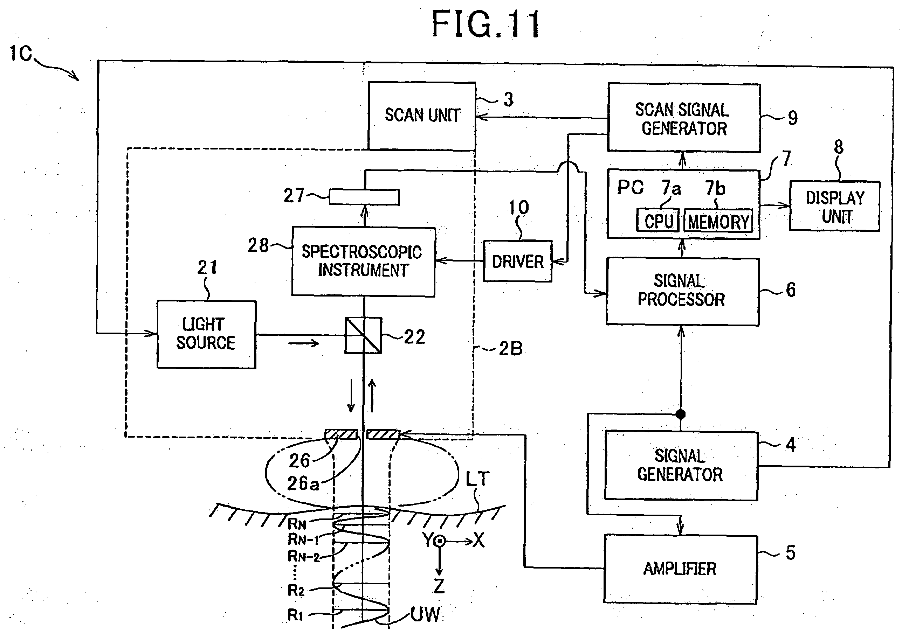

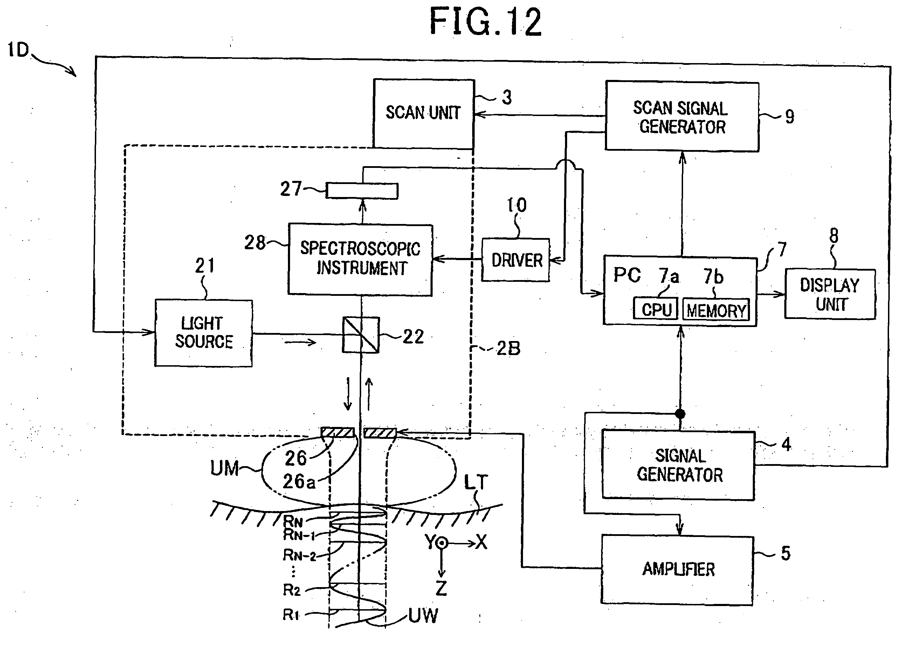

Referring to Figs. 11 and 12, a second embodiment of the present invention will now be described.

Fig. 11 outlines a biological observation apparatus according to the second embodiment of the present invention.

As shown in Fig. 11, the biological observation apparatus 1C comprises a radiation/reception unit 2B and a scan unit 3. The radiation/reception unit 2B is able to radiate ultrasound wave and light toward a living tissue LT functioning as an object to be examined and receive object light (reflected light) reflected from the living tissue LT in response to radiating the light. The scan unit 3 is configured to respond to a scan signal provided from the scan signal generator 9 so as to change the position (i.e., the scan position) of the radiation/reception unit 2B, during which both the ultrasound wave and the light are radiated, position by position. In the similar manner to the foregoing, the biological observation apparatus 1C comprises the signal generator 4, amplifier 5, signal processor 6, personal computer (PC) 7, display unit 8, scan signal generator 9, and driver 10.

The radiation/reception unit 2B comprises a spectroscopic instrument 28, in addition to the light source 21, the half mirror 22, the ultrasound transducer 26, and the light detector 27.

The driver 10 is configured to be in synchronization with the scan signal from the scan signal generator 9 to output a drive signal for driving the spectroscopic instrument 28.

The spectroscopic instrument 28 is placed between the half mirror 22 and the light detector 27 and is provided with either an acoustooptic device and a liquid crystal tunable filter, for example. In addition, this instrument 28 operates responsively to the a drive signal coming from the driver 10 to sweep its transmissive wavelength so as to apply wavelength decomposition to the object light transmitted from the half mirror 22. The wavelength-decomposed light is sent to the light detector 27, where the wavelength-decomposed light is detected to produce an electric spectral signal showing the wavelength-decomposed results and spectral signal is outputted to the signal processor 6. Additionally the spectroscopic instrument 28 outputs a wavelength decomposition signal to the signal processor 6 via the light detector 27.

The operations of the biological observation apparatus 1C according to the present embodiment will now be described.

First of all, an operator powers up each part of the biological observation apparatus 1C, and positions the ultrasound transducer 26 of the radiation/reception unit 2B such that the ultrasound wave and light are radiated in the Z-axis direction shown in Fig. 11 (i.e., the depth direction of the living tissue LT). Concurrently, a space between the ultrasound transducer 26 and the living tissue LT is filled with the ultrasound transmissive medium UM.

The operator then turns on switches, which are mounted in a not-shown operation device, to issue a command for radiating the ultrasound wave from the ultrasound transducer 26 to the living tissue LT.

In response to the command, the signal generator 4 generates a drive signal to the ultrasound transducer 26 by way of the amplifier 5, where the drive signal is for radiating, toward the living tissue TL, predetermined ultrasound waves having a waveform shown in Fig. 2A, for example.

The ultrasound wave emitted from the ultrasound transducer 26 causes the living tissue LT to maximize its density at the Z-axial positions in the living tissue LT, where such positions correspond to the maximized points (timings) of the ultrasound wave transmitted into the living tissue LT. The Z-axial positions, that is, the Z-axis local portions whose densities have been instantaneously maximized are illustrated as ultrasound wave fronts $R_1$, $R_2$, ..., $R_N$ in Fig. 11.

Meanwhile, at a timing when the ultrasound wave fronts RN are caused within the living tissue LT, that is, at a timing immediately after the completion of radiation of the ultrasound wave at a single scan position, a timing signal is given to the light source 21 of the radiation/reception unit 2B.

In response to the timing signal, a pulsed light is radiated from the light source 21 toward the half mirror 22. This radiated light has a frequency $f_L$ and is reflected by the half mirror 22 to be radiated through the opening 26a of the ultrasound transducer 26 in the Z-axis direction (the depth direction in the living tissue LT). In the living tissue LT, the radiated light encounters each of the plurality of ultrasound wave fronts $R_1$, $R_2$, ..., $R_N$, resulting in that the light is partly reflected by each wave front. The reflected pulsed light, which is from each of the ultrasound wave fronts $R_1$, $R_2$, ..., $R_N$, is returned to the spectroscopic instrument 28 via the opening 26a and the half mirror 22 as an object light (reflected light) that has been subjected to Doppler shift (i.e., frequency modulation) of a frequency $f_{d1}$ (, $f_{d2}$, ..., $f_{dN}$).

In the spectroscopic instrument 28, the object light undergoes the wavelength decomposition, and its wavelength decomposed light is detected by the light detector 27 and converted to electric spectral signals, which is sent to the signal processor 6.

In the signal processor 6, based on the drive signal given from the signal generator 4, the spectral distribution of the ultrasound wave emitted from the ultrasound transducer 26 is calculated, where the spectral distribution is shown for example at frequencies $f_{us1}$, $f_{us2}$, ..., $f_{usN}$. Additionally, in the signal processor 6, the spectral signals given from the light detector 27 are used to calculate the spectral distribution of the object light, where the spectral distribution is shown for example at frequencies $f_L$-$f_{d1}$, $f_L$-$f_{d2}$, ..., $f_L$-$f_{dN}$.

Further, in the signal processor 6, the spectral distribution of the object light and the frequency $f_L$ of the radiated light are used to calculate frequencies $f_{d1}$, $f_{d2}$, ..., $f_{dN}$ indicating Doppler shift amounts (frequency modulated amounts). Then, calculated are frequency ratios "$f_{d1}/f_{us1}$, $f_{d2}/f_{us2}$, ..., $f_{dN}/f_{usN}$" serving as scattering components of the object light and absorption components which correspond to intensities of the scattering components of the object light. Therefore, at an operator's desired observation region, it is possible to the signal processor 6 to acquire, at the same time, living-body information from N-piece local portions in the Z-axis direction (i.e., the depth direction).

In the PC 7, the CPU 7a uses the information of the scattering components and absorption components, which are given from the signal processor 6, to produce image data thereon. The produced image data are then stored into the memory 7b, where the produced image data are made to be related to scan positional information showing positions within a range to be scanned by the scan unit 3. When the PC 7 detects that the current scan position is not the end position thereof, the scan position is changed in any of the X-axis and Y-axis directions, and both the ultrasound wave and the light are radiated in a controlled manner. This control is done by the scan signal generator 9 under the control of the PC 7.

On the other hand, when the PC 7 detects that the current scan position is the end scan position, i.e., image data for one frame have been accumulated in the memory 7b, the PC 7 converts the image data to video signals and provides the display unit 8 with the video signals. Hence the display unit 8 displays a tomographic image of the living tissue LT which is located within the operator's desired body observation portion.

As stated, the biological observation apparatus 1C according to the present embodiment is able to acquire, at a time, at each scan position, the scattering components and/or the absorption components at the depth-directional N-piece local portions inside the living tissue LT. Hence, in the similar manner as the foregoing embodiment and modifications, it is possible to observe changes in the internal state of a desired portion of the object in an easier and faster manner.

As a modification, the radiation/reception unit 2B may be provided with means for converging the light being emitted to the living body LT. Such a means is for example a lens whose numeric aperture is small and this lens is placed between the half mirror 22 and the ultrasound transducer 26.

Furthermore, in the embodiment, the ultrasound wave and the light both are in parallel to the Z-axis direction, but this is not always a definitive structure. For example, any one of the two radiations may be oblique to the Z-axis direction.

<Third modification>

A modification of the foregoing biological observation apparatus 1C will now be described.

A biological observation apparatus 1D shown in Fig. 12 is modified from that shown in Fig. 11 in that the processes carried out by the signal processor 6 in Fig. 11 are carried out alternatively by the PC 7.

Hence, as shown in Fig. 12, the biological observation apparatus 1D has no signal processor. The spectral signal from the light detector 27 and the wavelength dissolution signal from the spectroscopic instrument 28 are sent to the PC 7.

Assume that DC (direct current) components of the spectral signal are denoted as $I_{DC,1}$, $I_{DC,2}$, ..., $I_{DC,N}$ and AC (alternating current) component amplitudes are denoted as $I_{AC,1}$, $I_{AC,2}$, ..., $I_{AC,N}$. In this case, a light intensity $I_1(t)$ based on the spectral signal can be shown by the following formula (3):

$$
\begin{aligned}
I_1(t) = {} & I_{DC,1} + I_{AC,1} \cos\left[2\pi(f_L - f_{d1})t + \phi_1\right] \\
& + I_{DC,2} + I_{AC,2} \cos\left[2\pi(f_L - f_{d2})t + \phi_2\right] \\
& + \ldots \\
& + I_{DC,N} + I_{AC,N} \cos\left[2\pi(f_L - f_{dN})t + \phi_N\right] \quad \cdots \quad (3)\ ,
\end{aligned}
$$

wherein $\phi$ depicts a phase difference.

The CPU 7a of the PC 7 applies Fourier transformation to the light intensity $I_1(t)$ shown by the formula (3), so that the following formula (4) can be obtained:

$$
\begin{aligned}
F_1(\omega) = {} & \int_{-T}^{+T}\left\{\sum_{i=1}^{N} I_{DC,i} + I_{AC,i}\cos((\omega_L + \omega_i)\,t + \phi_i)\right\}e^{-j\omega t}dt \\
= {} & \sum_{i=1}^{N}\frac{2 I_{DC,i}\sin \omega T}{\omega} + \sum_{i=1}^{N} I_{AC,i}\cos\phi\left\{\frac{\sin(\omega_L + \omega_i - \omega)T}{(\omega_L + \omega_i - \omega)} + \frac{\sin(\omega_L + \omega_i + \omega)T}{(\omega_L + \omega_i + \omega)}\right\} \\
& + j\sum_{i=1}^{N} I_{AC,i}\sin\phi\left\{\frac{\sin(\omega_L + \omega_i - \omega)T}{(\omega_L + \omega_i - \omega)} + \frac{\sin(\omega_L + \omega_i + \omega)T}{(\omega_L + \omega_i + \omega)}\right\} \quad \cdots \quad (4)\ ,
\end{aligned}
$$

wherein $\omega_i = 2\pi f_{di}$ and $\omega_L = 2\pi f_L$.

The CPU 7a, which also functions as a light spectrum acquiring member, extracts real-number components (i.e., intensity) in the formula (4) as values showing the spectral distribution of the object light.

Then the CPU 7a also functions as a calculator, where, on the basis of the spectral distribution of the object light and the frequency fL of the light, frequencies $f_{d1}$, $f_{d2}$, ..., $f_{dN}$ indicating Doppler shift amounts are calculated. Further, the CPU 7a uses the frequencies $f_{d1}$, $f_{d2}$, ..., $f_{dN}$ and the frequencies of the ultrasound wave from the ultrasound transducer 26, $f_{us1}$, $f_{us2}$, ..., $f_{usN}$, to calculate the ratios $f_{d1}/f_{us}1$, $f_{d2}/f_{us2}$, ..., $f_{dn}/f_{usN}$ serving as the scattering components of the object light. In addition, the CPU 7a calculates the intensities at specific positions, defined by those frequency ratios, as the absorption components of the object light.

In this way, at an operator's desired portion to be observed, it is possible to acquire, at a time, pieces of body information from N-piece local portions in the Z-axis direction within the living body LT. That is, the biological observation apparatus ID shown in Fig. 12 also obtains the similar advantages to those gained by the foregoing apparatus 1C. In addition, this apparatus 1D has no signal processor, thereby simplifying the apparatus compared to the foregoing apparatus 1A.

<Fourth modification>

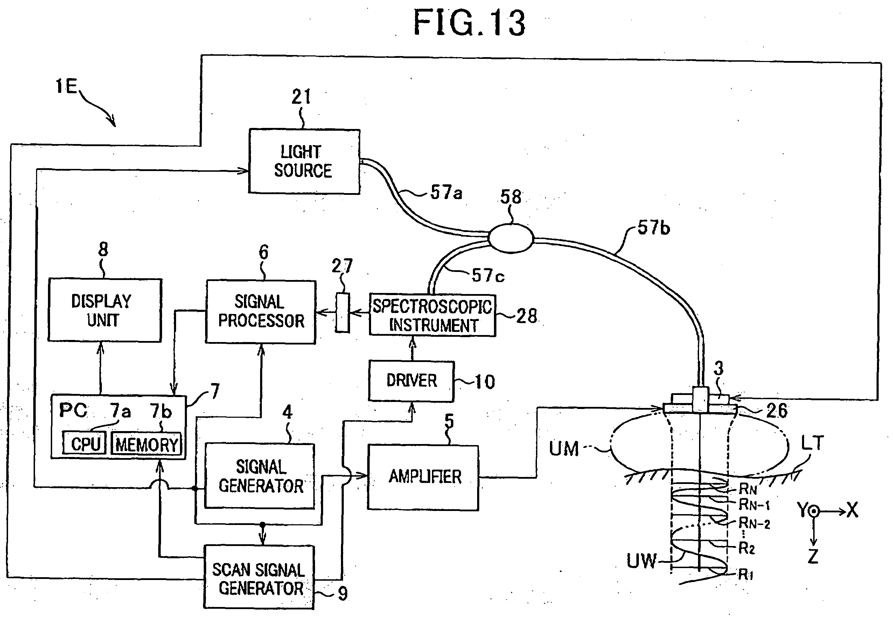

Referring to Fig. 13, a modification of the foregoing biological observation apparatus 1C will now be described. Fig. 13 shows a modified biological observation apparatus 1E provided with optical fibers and an optical coupler.

Practically, the biological observation apparatus 1E is provided, as its essential parts, optical fibers 57a to 57c and an optical coupler 58 serving as a light-receiving member, in addition to the scan converter 3, signal generator 4, amplifier 5, signal processor 6, PC7, display unit 8, scan signal generator 9, driver 10, light source 21, ultrasound transducer 26, light detector 27, and spectroscopic instrument 28.

As shown in Fig. 13, the optical fiber 57a has one end connected to the light source 21 and the other end connected to the optical coupler 58. The optical fiber 57b contains a light-sending fiber bundle and a light-receiving fiber bundle and has one end connected to the optical coupler 58 and the other end connected to an opening (for example, though not shown in Fig. 8, the opening 26a) formed at the center of the ultrasound transducer 26 in such a manner that the end is inserted therethrough.

The last optical fiber 57c has one end connected to the optical coupler 58 and the other end connected to the spectroscopic instrument 28.

Thus, in this biological observation apparatus 1E, the light emitted from the light source 21 is radiated toward the living tissue LT via the optical fiber 57a, the optical coupler 58, and the light-sending fiber bundle of the optical fiber 57b.

The light radiated into the living tissue LT is partly reflected in sequence at the respective ultrasound wave fronts $R_1$, $R_2$, .... $R_N$ produced therein, where the object light (reflected light) subjected to Doppler shift (frequency modulation) of frequencies $f_{d1}$, $f_{d2}$, ..., $f_{dN}$ is generated as described. This object light is made to enter the light-receiving fiber bundle of the optical fiber 57b, and then sent to the spectroscopic instrument 28 via the optical coupler 58 and the optical fiber 57c.

After this, the object light is subjected to the processes which are the same or similar to those carried out in the biological observation apparatus 1C. Accordingly, the biological observation apparatus 1E shown in Fig. 13 is also able to have the advantages similar to the foregoing one.

Incidentally, in each of the biological observation apparatuses shown in Figs. 11, 12 and 13, the spectroscopic instrument 28 is not always limited to use of the acoustooptic device or the liquid crystal tunable filter, but may be provided with the use of, for example, a diffraction grating. In such a case, the driver 10 may be omitted from the configuration.

Although the description above contains many specificities, these should not be construed as limiting the scope of the disclosure but as merely providing illustrations of some of the presently preferred embodiments of the present invention.

**Claims**

1.  A medical apparatus (1, 1A-E) comprising:

    an ultrasound radiating member (26);
    a signal generator (4) adapted to produce a drive signal for driving the ultrasound radiating member (26);
    the ultrasound radiating member (26) being adapted to, in response to the drive signal from the signal generator (4), to radiate an ultrasound wave into an object (LT) to be examined, the ultrasound wave having a plurality of frequency components different from each other; and
    a light radiating member (21) adapted to radiate light into a region within the object (LT), at a predetermined time immediately after the completion of radiation of the ultrasound wave, such that the ultrasound wave has already been radiated into the region at said predetermined time;
    **characterized by** a light receiving member (27, 28) adapted to receive light reflected and scattered in the region in response to the radiated light from the light radiating member (21); and
    a calculator (6, 7) configured to:

        receive as an input:

            a spectral signal for providing a spectral distribution of the light received by the light receiving member (27, 28); and

the drive signal for providing a spectral distribution of the ultrasound wave radiated by the ultrasound radiating member (26),

calculate a Doppler shift amount $f_{d1}$, $f_{d2}$, ... $f_{dN}$ in a frequency of the reflected light indicative of a reflected and a scattered state of the radiated light in the region based on the spectral distribution of the light received by the light receiving member (27, 28);
calculate scattering components of the reflected light based on said spectral distribution of the light received by said light receiving member (27, 28) and said spectral distribution of the ultrasound wave radiated by the ultrasound radiating member (26), said spectral distribution having frequencies $f_{us1}$, $f_{us2}$, ... $f_{usN}$, wherein the frequency ratios $f_{d1/}$ $f_{us1}$, $f_{d2/}$ $f_{us2}$, ...
$f_{dN}/f_{usN}$ serve as said scattering components; and
calculate absorption components corresponding to intensities of the scattering components at each frequency ratio.

2. The medical apparatus according to claim 1, wherein the ultrasound radiating member (26) comprises an ultrasound-wave generator that is adapted to generate, as the ultrasound wave having the plurality of frequency components different from each other, an ultrasound wave whose frequency changes as time elapses.

3. The medical apparatus according to claim 2, wherein the ultrasound wave having the plurality of frequency components different from each other is a pulsed ultrasound wave.

4. The medical apparatus according to claim 2 or 3, wherein the ultrasound wave having the plurality of frequency components different from each other is a continuous ultrasound wave, the frequency of which increases or decreases.

5. The medical apparatus according to claim 1, wherein the light radiating member (21) is configured to radiate, as the light, pulsed light when the ultrasound radiating member (26) completes the radiation of the ultrasound wave.

6. The medical apparatus according to claim 1, wherein the light radiating member (21) is configured to radiate, as the light, continuous light.

7. The medical apparatus according to claim 1, wherein the ultrasound wave radiated from the ultrasound radiating member (26) is radiated along a light axis along which the light is radiated from the light radiating member (21).

8. The medical apparatus according to claim 2, wherein the ultrasound wave is an ultrasound wave, the frequency of which becomes higher as time elapses.

9. The medical apparatus according to claim 8, comprising:

determination means for determining a timing at which the ultrasound radiating member completes, by one time, the radiation of the ultrasound wave at a desired single position on the object; and
command means for commanding the light radiating member (21) to radiate the light at the desired position on the object (LT) at the predetermined time, when the determination means determines the timing.

10. The medical apparatus according to claim 9, comprising means for changing the position on the object, and adapted to subject said position on the object to the radiation from the ultrasound radiating member (26) and the light radiating member (21), when the light radiating member (21) completes the radiation of the light.

11. The medical apparatus according to claim 10, wherein the ultrasound radiating member (26) and the light radiating member (21) are incorporated in a single unit (2).

12. The medical apparatus according to claim 11, wherein the ultrasound wave radiated from the ultrasound radiating member (26) and the light radiated from the light radiating member (21) are radiated in a same radiation direction and on a same radiated position on the object.

13. The medical apparatus according to any preceding claim, comprising a presentation unit (8) that visually presents the information calculated by the calculator (6, 7).

**14.** A method of detecting information indicative of an internal state of an object to be examined, comprising:

radiating, in response to a drive signal, an ultrasound wave into the object (LT), the ultrasound wave having a plurality of frequency components different from each other; radiating light into a region within the object (LT), at a predetermined time immediately after the completion of radiation of the ultrasound wave, such that the ultrasound wave has already been radiated into the region (R) at said predetermined time; and receiving light reflected and scattered in the region in response to the radiated light, **characterized by** further comprising the steps of:

receiving as an input:

a spectral signal for providing a spectral distribution of the received light and the drive signal for providing the a spectral distribution of the radiated ultrasound wave,

calculating a Doppler shift amount $f_{d1}$, $f_{d2}$, ... $f_{dN}$ in a frequency of the reflected light indicative of a reflected and a scattered state of the radiated light in the region based on the spectral distribution of the light received by the light receiving member (27, 28);
calculating scattering components of the reflected light based on said spectral distribution of the light received by said light receiving member (27, 28) and said spectral distribution of the ultrasound wave radiated by the ultrasound radiating member (26), the spectral distribution of the ultrasound wave having frequencies $f_{us1}$, $f_{us2}$, ... $f_{usN}$, wherein the frequency ratios $f_{d1}/f_{us1}$, $t_{d2}/f_{us2}$, .... $f_{dN}/f_{usN}$ serve as said scattering components; and
calculating absorption components corresponding to intensities of the scattering components at each frequency ratio.

**15.** The method according to claim 14, wherein the ultrasound wave changes a frequency thereof as time elapses and the ultrasound wave and the light are repeatedly radiated at different positions on the object, the different positions being subjected to the radiation of both the ultrasound wave and the light.

**Patentansprüche**

**1.** Medizinische Vorrichtung (1, 1A-E) mit:

einem Ultraschallstrahlungselement (26);
einem Signalgenerator (4), der dazu ausgebildet ist, ein Treibersignal zum Treiben des Ultraschallstrahlungselements (26) zu erzeugen;
wobei das Ultraschallstrahlungselement (26) dazu ausgebildet ist, als Reaktion auf das Treibersignal von dem Signalgenerator (4) eine Ultraschallwelle in ein zu untersuchendes Objekt (LT) zu strahlen, wobei die Ultraschallwelle eine Mehrzahl von Frequenzkomponenten aufweist, die sich voneinander unterscheiden; und
einem Lichtstrahlungselement (21), das dazu ausgebildet ist, Licht zu einer vorgegebenen Zeit unmittelbar nach der Beendigung der Strahlung der Ultraschallwelle in einen Bereich innerhalb des Objektes (LT) zu strahlen, so dass die Ultraschallwelle zu der vorgegebenen Zeit bereits in den Bereich gestrahlt wurde;
**gekennzeichnet durch** ein Lichtempfangselement (27, 28), das dazu ausgebildet ist, in dem Bereich reflektiertes und gestreutes Licht als Reaktion auf das von dem Lichtstrahlungselement (21) ausgestrahlte Licht zu empfangen; und
eine Recheneinrichtung (6, 7), die dazu ausgebildet ist:

als eine Eingabe zu empfangen:

ein Spektralsignal zum Vorsehen einer spektralen Verteilung des von dem Lichtempfangselement (27, 28) empfangenen Lichtes; und
das Treibersignal zum Vorsehen einer spektralen Verteilung der von dem Ultraschallstrahlungselement (26) ausgestrahlten Ultraschallwelle,
einen Dopplerverschiebungsbetrag $f_{d1}$, $f_{d2}$, ...$f_{dN}$ in einer Frequenz des reflektierten Lichtes, der einen reflektierten und gestreuten Zustand des ausgestrahlten Lichtes in dem Bereich anzeigt, auf Grundlage der spektralen Verteilung des von dem Lichtempfangselement (27, 28) empfangenen Lichtes zu berechnen;

Streukomponenten des reflektierten Lichtes auf Grundlage der spektralen Verteilung des von dem Lichtempfangselement (27, 28) empfangenen Lichtes und der spektralen Verteilung der von dem Ultraschallstrahlungselement (26) ausgestrahlten Ultraschallwelle zu berechnen, wobei die spektrale Verteilung Frequenzen

$f_{us1}$, $f_{us2}$, ...$f_{usN}$, hat und wobei die Frequenzverhältnisse $f_{d1}/f_{us1}$,
$f_{d2}/f_{us2}$, ...$f_{dN}/f_{usN}$ als die Streukomponenten dienen; und
Absorptionskomponenten zu berechnen, die Intensitäten der Streukomponenten bei jedem Frequenzverhältnis entsprechen.

2. Medizinische Vorrichtung nach Anspruch 1, wobei das Ultraschallstrahlungselement (26) einen Ultraschallwellengenerator aufweist, der dazu ausgebildet ist, als die Ultraschallwelle mit der Mehrzahl von Frequenzkomponenten, die sich voneinander unterscheiden, eine Ultraschallwelle zu erzeugen, deren Frequenz sich mit dem Ablauf von Zeit ändert.

3. Medizinische Vorrichtung nach Anspruch 2, wobei die Ultraschallwelle mit der Mehrzahl von Frequenzkomponenten, die sich voneinander unterscheiden, eine gepulste Ultraschallwelle ist.

4. Medizinische Vorrichtung nach Anspruch 2 oder 3, wobei die Ultraschallwelle mit der Mehrzahl von Frequenzkomponenten, die sich voneinander unterscheiden, eine Dauerultraschallwelle ist, deren Frequenz zunimmt oder abnimmt.

5. Medizinische Vorrichtung nach Anspruch 1, wobei das Lichtstrahlungselement (21) dazu ausgebildet ist, als das Licht gepulstes Licht auszustrahlen, wenn das Ultraschallstrahlungselement (26) die Ausstrahlung der Ultraschallwelle beendet.

6. Medizinische Vorrichtung nach Anspruch 1, wobei das Lichtstrahlungselement (21) dazu ausgebildet ist, als das Licht Dauerlicht auszustrahlen.

7. Medizinische Vorrichtung nach Anspruch 1, wobei die von dem Ultraschallstrahlungselement (26) ausgestrahlte Ultraschallwelle entlang einer Lichtachse ausgestrahlt wird, entlang derer das Licht von dem Lichtstrahlungselement (21) ausgestrahlt wird.

8. Medizinische Vorrichtung nach Anspruch 2, wobei die Ultraschallwelle eine Ultraschallwelle ist, deren Frequenz sich mit dem Ablauf von Zeit erhöht.

9. Medizinische Vorrichtung nach Anspruch 8 mit:

   einer Bestimmungseinrichtung zum Bestimmen einer Zeit, zu der das Ultraschallstrahlungselement bis zu einer Zeit die Ausstrahlung der Ultraschallwelle an einer erwünschten einzelnen Position des Objektes beendet; und einer Befehlseinrichtung zum Erteilen eines Befehls an das Lichtstrahlungselement (21), das Licht zu der vorgegebenen Zeit an der erwünschten Position an dem Objekt (LT) auszustrahlen, wenn die Bestimmungseinrichtung die Zeit bestimmt.

10. Medizinische Vorrichtung nach Anspruch 9 mit einer Einrichtung zum Ändern der Position an dem Objekt, die dazu ausgebildet ist, die Position an dem Objekt der Strahlung von dem Ultraschallstrahlungselement (26) und dem Lichtstrahlungselement (21) auszusetzen, wenn das Lichtstrahlungselement (21) die Ausstrahlung des Lichtes beendet .

11. Medizinische Vorrichtung nach Anspruch 10, wobei das Ultraschallstrahlungselement (26) und das Lichtstrahlungselement (21) in eine einzelne Einheit (2) integriert sind.

12. Medizinische Vorrichtung nach Anspruch 11, wobei die von dem Ultraschallstrahlungselement (26) ausgestrahlte Ultraschallwelle und das von dem Lichtstrahlungselement (21) ausgestrahlte Licht in eine gleiche Strahlungsrichtung und an eine gleiche angestrahlte Position an dem Objekt gestrahlt werden.

13. Medizinische Vorrichtung nach einem der vorhergehenden Ansprüche mit einer Darstellungseinheit (8), die die von der Recheneinrichtung (6, 7) berechnete Information darstellt.

**14.** Verfahren zum Erfassen von Informationen, die einen internen Zustand eines zu untersuchenden Objektes anzeigt, das beinhaltet:

Strahlen, als Reaktion auf ein Treibersignal, einer Ultraschallwelle in das Objekt (LT), wobei die Ultraschallwelle eine Mehrzahl von Frequenzkomponenten hat, die sich voneinander unterscheiden; Strahlen von Licht in einen Bereich innerhalb des Objektes (LT) zu einer vorgegebenen Zeit unmittelbar nach Beendigung der Strahlung der Ultraschallwelle, so dass die Ultraschallwelle zu der vorgegebenen Zeit bereits in den Bereich (R) gestrahlt wurde; und

Empfangen von in dem Bereich reflektiertem Licht als Reaktion auf das ausgestrahlte Licht, **dadurch gekennzeichnet, dass** das Verfahren ferner folgende Schritte beinhaltet:

Empfangen als eine Eingabe:

eines Spektralsignals zum Vorsehen einer spektralen Verteilung des empfangenen Lichtes und des Treibersignals zum Vorsehen einer spektralen Verteilung der ausgestrahlten Ultraschallwelle, Berechnen eines Dopplerverschiebungsbetrags $f_{d1}$, $f_{d2}$, ...$f_{dN}$ in einer Frequenz des reflektierten Lichtes, der einen reflektierten und gestreuten Zustand des ausgestrahlten Lichtes in dem Bereich anzeigt, auf Grundlage der spektralen Verteilung des von dem Lichtempfangselement (27, 28) empfangenen Lichtes;

Berechnen von Streukomponenten des reflektierten Lichtes auf Grundlage der spektralen Verteilung des von dem Lichtempfangselement (27, 28) empfangenen Lichtes und der spektralen Verteilung der von dem Ultraschallstrahlungselement (26) ausgestrahlten Ultraschallwelle, wobei die spektrale Verteilung der Ultraschallwelle Frequenzen $f_{us1}$, $f_{us2}$, ... $f_{usN}$ hat und wobei die Frequenzverhältnisse $f_{d1}/f_{us1}$, $f_{d2}/f_{us2}$ ... $f_{dN}/f_{usN}$ als die Streukomponenten dienen; und

Berechnen von Absorptionskomponenten, die Intensitäten der Streukomponenten bei jedem Frequenzverhältnis entsprechen.

**15.** Verfahren nach Anspruch 14, wobei die Ultraschallwelle eine Frequenz davon mit dem Ablauf von Zeit ändert und die Ultraschallwelle und das Licht wiederholt an verschiedene Positionen an dem Objekt gestrahlt werden, wobei die unterschiedlichen Positionen der Strahlung sowohl der Ultraschallwelle als auch des Lichtes ausgesetzt werden.


**Revendications**

**1.** Appareil médical (1, 1A-E) comprenant :

un élément (26) de rayonnement d'ultrasons ;
un générateur (4) de signal adapté à produire un signal d'entraînement pour entraîner l'élément (26) de rayonnement d'ultrasons ;
l'élément (26) de rayonnement d'ultrasons étant adapté à, en réponse au signal d'entraînement provenant du générateur (4) de signal, rayonner une onde ultrasonore jusque dans un objet (LT) à examiner, l'onde ultrasonore ayant une pluralité de composantes de fréquence différentes les unes des autres ; et
un élément (21) de rayonnement de lumière adapté à rayonner une lumière jusque dans une région à l'intérieur de l'objet (LT), à un moment prédéterminé immédiatement après la fin du rayonnement de l'onde ultrasonore, de telle sorte que l'onde ultrasonore a déjà été rayonnée jusque dans la région audit moment prédéterminé ; **caractérisé par** un élément (27, 28) de réception de lumière adapté à recevoir une lumière réfléchie et diffusée dans la région en réponse à la lumière rayonnée provenant de l'élément (21) de rayonnement de lumière ; et un calculateur (6, 7) configuré pour :

recevoir comme une entrée :

un signal spectral pour fournir une distribution spectrale de la lumière reçue par l'élément (27, 28) de réception de lumière ; et
le signal d'entraînement pour fournir une distribution spectrale de l'onde ultrasonore rayonnée par l'élément (26) de rayonnement d'ultrasons,

calculer une quantité de décalage Doppler $f_{d1}$, $f_{d2}$, ... $f_{dN}$ dans une fréquence de la lumière réfléchie indicatrice d'un état réfléchi et d'un état diffusé de la lumière rayonnée dans la région sur la base de la distribution spectrale de la lumière reçue par l'élément (27, 28) de réception de lumière ;

calculer des composantes de diffusion de la lumière réfléchie sur la base de ladite distribution spectrale de la lumière reçue par ledit élément (27, 28) de réception de lumière et de ladite distribution spectrale de l'onde ultrasonore rayonnée par l'élément (26) de rayonnement d'ultrasons, ladite distribution spectrale ayant des fréquences $f_{us1}$, $f_{us2}$,... $t_{usN}$, dans lequel les rapports de fréquences $f_{d1}/f_{us1}$, $f_{d2}/f_{us2}$, ... $f_{dN}/f_{usN}$ servent comme lesdites composantes de diffusion ; et

calculer des composantes d'absorption correspondant à des intensités des composantes de diffusion à chaque rapport de fréquences.

2. Appareil médical selon la revendication 1, dans lequel l'élément (26) de rayonnement d'ultrasons comprend un générateur d'onde ultrasonore qui est adapté à générer, comme l'onde ultrasonore ayant la pluralité de composantes de fréquence différentes les unes des autres, une onde ultrasonore dont la fréquence change alors que le temps s'écoule.

3. Appareil médical selon la revendication 2, dans lequel l'onde ultrasonore ayant la pluralité de composantes de fréquence différentes les unes des autres est une onde ultrasonore pulsée.

4. Appareil médical selon la revendication 2 ou 3, dans lequel l'onde ultrasonore ayant la pluralité de composantes de fréquence différentes les unes des autres est une onde ultrasonore continue, la fréquence de laquelle augmente ou diminue.

5. Appareil médical selon la revendication 1, dans lequel l'élément (21) de rayonnement de lumière est configuré pour rayonner, comme la lumière, une lumière pulsée lorsque l'élément (26) de rayonnement d'ultrasons termine le rayonnement de l'onde ultrasonore.

6. Appareil médical selon la revendication 1, dans lequel l'élément (21) de rayonnement de lumière est configuré pour rayonner, comme la lumière, une lumière continue.

7. Appareil médical selon la revendication 1, dans lequel l'onde ultrasonore rayonnée depuis l'élément (26) de rayonnement d'ultrasons est rayonnée le long d'un axe de lumière le long duquel la lumière est rayonnée depuis l'élément (21) de rayonnement de lumière.

8. Appareil médical selon la revendication 2, dans lequel l'onde ultrasonore est une onde ultrasonore, la fréquence de laquelle devient plus élevée alors que le temps s'écoule.

9. Appareil médical selon la revendication 8, comprenant :

un moyen de détermination pour déterminer un instant auquel l'élément de rayonnement d'ultrasons termine, en une fois, le rayonnement de l'onde ultrasonore sur une position unique désirée sur l'objet ; et

un moyen de commande pour commander à l'élément (21) de rayonnement de lumière de rayonner la lumière sur la position désirée sur l'objet (LT) au moment prédéterminé, lorsque le moyen de détermination détermine l'instant.

10. Appareil médical selon la revendication 9, comprenant un moyen pour changer la position sur l'objet, et adapté à soumettre ladite position sur l'objet au rayonnement provenant de l'élément (26) de rayonnement d'ultrasons et de l'élément (21) de rayonnement de lumière, lorsque l'élément (21) de rayonnement de lumière termine le rayonnement de la lumière.

11. Appareil médical selon la revendication 10, dans lequel l'élément (26) de rayonnement d'ultrasons et l'élément (21) de rayonnement de lumière sont incorporés en une unité (2) unique.

12. Appareil médical selon la revendication 11, dans lequel l'onde ultrasonore rayonnée depuis l'élément (26) de rayonnement d'ultrasons et la lumière rayonnée depuis l'élément (21) de rayonnement de lumière sont rayonnées dans un même sens de rayonnement et sur une même position rayonnée sur l'objet.

13. Appareil médical selon une quelconque revendication précédente, comprenant une unité (8) de présentation qui présente visuellement les informations calculées par le calculateur (6, 7).

**14.** Procédé de détection d'informations indicatrices d'un état interne d'un objet à examiner, comprenant :

le rayonnement, en réponse à un signal d'entraînement, d'une onde ultrasonore jusque dans l'objet (LT), l'onde ultrasonore ayant une pluralité de composantes de fréquence différentes les unes des autres ; le rayonnement d'une lumière jusque dans une région à l'intérieur de l'objet (LT), à un moment prédéterminé immédiatement après la fin du rayonnement de l'onde ultrasonore, de telle sorte que l'onde ultrasonore a déjà été rayonnée jusque dans la région (R) audit moment prédéterminé ; et
la réception d'une lumière réfléchie et diffusée dans la région en réponse à la lumière rayonnée, **caractérisé en ce que** comprenant en outre les étapes de :

réception comme une entrée :

d'un signal spectral pour fournir une distribution spectrale de la lumière reçue et
du signal d'entraînement pour fournir une distribution spectrale de l'onde ultrasonore rayonnée,

calcul d'une quantité de décalage Doppler $f_{d1}$, $f_{d2}$, ... $f_{dN}$ dans une fréquence de la lumière réfléchie indicatrice d'un état réfléchi et d'un état diffusé de la lumière rayonnée dans la région sur la base de la distribution spectrale de la lumière reçue par l'élément (27, 28) de réception de lumière ;
calcul de composantes de diffusion de la lumière réfléchie sur la base de ladite distribution spectrale de la lumière reçue par ledit élément (27, 28) de réception de lumière et de ladite distribution spectrale de l'onde ultrasonore rayonnée par l'élément (26) de rayonnement d'ultrasons, la distribution spectrale de l'onde ultrasonore ayant des fréquences $f_{us1}$, $f_{us2}$, ... $f_{usN}$, dans lequel les rapports de fréquences $f_{d1}/f_{us1}$, $f_{d2}/t_{us2}$,... $f_{dN}/f_{usN}$ servent comme lesdites composantes de diffusion ; et
calcul de composantes d'absorption correspondant à des intensités des composantes de diffusion à chaque rapport de fréquences.

**15.** Procédé selon la revendication 14, dans lequel l'onde ultrasonore change une fréquence de celle-ci alors que le temps s'écoule et l'onde ultrasonore et la lumière sont rayonnées de façon répétée sur différentes positions sur l'objet, les différentes positions étant soumises au rayonnement à la fois de l'onde ultrasonore et de la lumière.

# FIG.1

EP 2 016 891 B1

# FIG.2A

# FIG.2B

# FIG.2C

# FIG.2D

INTENSITY $\quad (f_1 < f_2 < \cdots < f_{N-1} < f_N)$

$f_1 \qquad f_2 \qquad f_3 \qquad f_4 \qquad f_{N-1} \qquad f_N$

$\cdots$

TIME

$T \qquad T$

# FIG.3

INTENSITY

$\cdots$

$\cdots$

0 $\qquad\qquad$ FREQUENCY

$f_{d1}/f_{us1}$

$f_{d2}/f_{us2}$

$f_{d(N-2)}/f_{us(N-2)}$

$f_{d(N-1)}/f_{us(N-1)}$

$f_{dN}/f_{usN}$

# FIG.4

START

S1 — READ INFORMATION OF SCATTERING COMPONENTS AND/OR ABSORPTION COMPONENTS

S2 — PRODUCE AND STORE IMAGE DATA

S3 — END OF SCAN?

NO

S4 — COMMAND CHANGES OF SCAN POSITION

YES

S5 — OUTPUT IMAGE DATA TO DISPLAY UNIT

END

# FIG.5

# FIG.6

EP 2 016 891 B1

# FIG.7

INTENSITY

0

$-(f_{d1}/f_{us1})$    $f_{d1}/f_{us1}$    $f_{d3}/f_{us3}$    FREQUENCY

$f_{d2}/f_{us2}$

# FIG.8

EP 2 016 891 B1

# FIG.9

# FIG.10

# FIG.11

EP 2 016 891 B1

FIG.12

# FIG.13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000088743 A
- JP 10179584 A
- WO 2006090298 A1
- WO 9850781 A1
- WO 02059580 A1
- US 6738653 B1
- JP 2005224399 A

**Non-patent literature cited in the description**

- **L. V. WANG ; G. KU.** Frequency-swept ultrasound-modulated optical tomography of scattering media. *Optics Letters,* 1998, vol. 23 (12), 975-977